(19) Europäisches Patentamt
European Patent Office
Office européen des brevets

(11) EP 1 510 815 A1

(12) EUROPEAN PATENT APPLICATION
published in accordance with Art. 158(3) EPC

(43) Date of publication:
02.03.2005 Bulletin 2005/09

(21) Application number: 03746450.0

(22) Date of filing: 10.04.2003

(51) Int Cl.[7]: G01N 27/72

(86) International application number:
PCT/JP2003/004581

(87) International publication number:
WO 2003/087804 (23.10.2003 Gazette 2003/43)

(84) Designated Contracting States:
AT BE BG CH CY CZ DE DK EE ES FI FR GB GR HU IE IT LI LU MC NL PT RO SE SI SK TR

(30) Priority: 12.04.2002 JP 2002110706

(71) Applicant: Japan Science and Technology Agency
Kawaguchi-shi, Saitama 332-0012 (JP)

(72) Inventor: TAKAHASHI, Seiki
Morioka-shi, Iwate 020-0851 (JP)

(74) Representative:
Röthinger, Rainer, Dipl.-Phys. et al
c/o Wuesthoff & Wuesthoff
Patent- und Rechtsanwälte
Schweigerstrasse 2
81541 München (DE)

# (54) METHOD OF NONDESTRUCTIVE EXAMINATION OF CHROMIUM-CONTAINING NICKEL-BASED ALLOY FOR GRAIN BOUNDARY CORROSION AND EXAMINATION APPARATUS

(57) Discloses is a nondestructive inspection method of grain-boundary attack due to thermal sensitization in a chromium-containing nickel-based alloy, such as Inconel 600 alloy. The method comprises measuring a saturation magnetization $M_s(T_i)$ of a test piece at each of a plurality of measuring temperatures defined by equally dividing a given measuring temperature range in the range of a minimum to a maximum of Curie temperatures corresponding to respective chromium concentrations in a chromium impoverished region of the alloy, and then quantitatively determining an average spatial distribution of the chromium impoverished region of the test piece, or the chromium-concentration-specific volume of the chromium impoverished region adjacent to the crystal grain boundaries of the test piece, according to a given calculation formula. The present invention can solve disadvantages in a conventional method of inspecting a chromium impoverished region of a chromium-containing nickel-based alloy, such as destruction of the alloy surface caused by an etching or breaking operation, which is incongruous with the philosophy of a nondestructive inspection, and poor information about chromium impoverished region, which is obtainable only in the alloy surface.

FIG.10

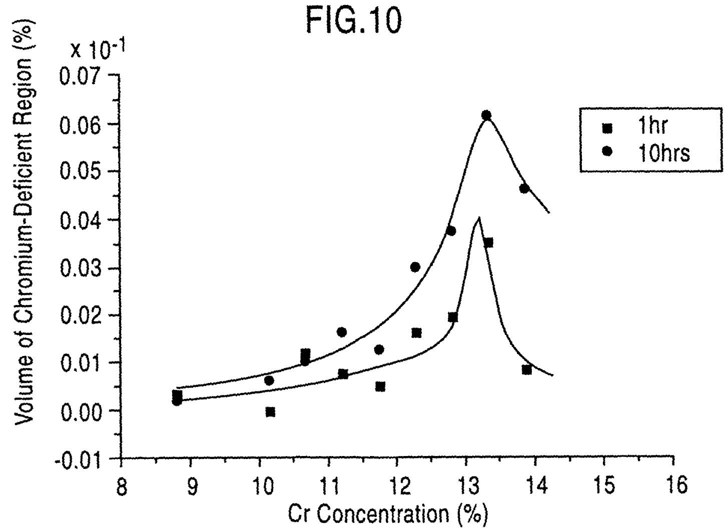


EP 1 510 815 A1

## Description

### TECHNICAL FIELD

The present invention relates to a method for nondestructive inspection of grain-boundary attack due to thermal sensitization in a chromium-containing nickel-based alloy as typified by Inconel alloys. The present invention also relates to an inspection apparatus for use in this method.

### BACKGROUND ART

Inconel alloys, which are typical ones of chromium-containing nickel-based alloys, are a heat-resistant alloy comprising major components of nickel and about 15 to 23 wt% of chromium, and some of the Inconel alloys additionally contain at least one of iron, cobalt and molybdenum. A typical one or Inconel 600 alloy (Ni: 76.0 wt%, Cr: 15.5 wt%, Fe: 7.8 wt%, Mn: 0.4 wt%, Si: 0.2 wt%, C: 0.08 wt%) is widely used in peripheral equipment of nuclear reactors, thermal plants, chemical plants, etc. In these cases, due to thermal sensitization caused by a heat treatment in a welding process or the like or a high-temperature condition maintained for a long time of period, chromium carbide precipitates are formed along the crystal grain boundaries of the alloy to thereby produce chromium-impoverished region. This chromium impoverished region is one of the factors causing stress corrosion cracking.
Heretofore, such stress corrosion cracking has been generally inspected through methods based on destructive tests, such as a method in which a test piece is set in an operating environment, and periodically taken out to subject it to etching using a chemical agent and optical-microscopic observation, a method in which chromium-carbide precipitates in a test piece are electrochemically etched and checked up, and a Charpy test.
For example, Japanese Patent Publication No. 02-054501 discloses an grain-boundary attack test method in which polarization is performed at a potential allowing nickel to cause active dissolution in an aqueous solution containing nitric acid, to detect a chromium impoverished region of a chromium-containing nickel-based alloy.
It has also been known to use a magnetic measurement method in detecting high-temperature aging embrittlement or strain damage in an actual member as an iron-based alloy product, such as ferrite-containing stainless steel or low-alloy steel (see, for example, Japanese Patent publication No. 07-006950 and Japanese Laid-Open Patent publication No. 04-218764).

### DISCLOSURE OF INVENTION

As mentioned above, a chromium-containing nickel-based alloy typified by the Inconel alloys is thermally sensitized to form chromium carbide precipitates in the vicinity of the crystal grain boundaries of the alloy and thereby produce a chromium impoverished region in the crystal grains. FIG. 1 schematically shows a chromium concentration distribution in the state after chromium carbide precipitates are formed in the vicinity of the crystal grain boundaries of the Inconel 600 alloy. For example, as illustrated, a portion having 10 wt% or less of chromium concentration is formed around the crystal grain boundary to cause a high risk of stress corrosion cracking. This chromium concentration distribution is caused by chromium carbide precipitates to be formed when a structural member of the Inconel 600 alloy is subjected to a heat treatment in a welding process, or held at a temperature of 600 to 700°C for a long time of period, and the pattern of the distribution is varied depending on the holding time.
The conventional method of inspecting a chromium impoverished region involves a destructive operation, such etching or breaking of the surface of a test piece as described above, which is incongruous with the philosophy of a nondestructive inspection, and can obtain information about the chromium impoverished region only in the surface of the test piece. Moreover, in the conventional inspection method, a great deal of time and effort has to be consumed to quantitatively determine chromium concentrations in the chromium impoverished region and the volume of the chromium impoverished region.
A conventional inspection method based on the measurement of magnetic susceptibility permits only qualitative observation of precipitates of chromium atoms and a chromium impoverished region in the vicinity of the crystal grain boundaries.
The aforementioned magnetic measurement method for a Fe-based alloy member is intended to calculate the variation of saturation magnetization based on the comparison with known data. Thus, the volume of a chromium impoverished region caused by the formation of chromium carbide precipitates in a chromium-containing nickel-based alloy cannot be determined in a divided manner on the basis of the chromium concentrations in chromium impoverished region.
The inventor has developed a nondestructive inspection method capable of quantitatively measuring the presence of a chromium impoverished region causing grain-boundary attack cracking in a chromium-containing nickel-based alloy, by use of a magnetic means, and an inspection apparatus for use in this method.

Specifically, the present invention provides a method for nondestructive inspection of grain-boundary attack due to thermal sensitization in a chromium-containing nickel-based alloy. The method comprises: measuring a saturation magnetization $M_s(T_i)$ of a test piece at each of a plurality of measuring temperatures defined by equally dividing a given measuring temperature range in the range of a minimum to a maximum of Curie temperatures corresponding to respective chromium concentrations in a chromium impoverished region of the alloy; and calculating νk according to the following formula (1) to quantitatively determine the volume of the chromium impoverished region in a divided manner on the basis of the chromium concentrations:

$$M_s(T_i) = \sum_{k=1}^{i} \frac{\nu_k M_k(T_i)}{V} \qquad (1),$$

wherein: νk is the volume of the chromium impoverished region having a chromium concentration $C_k$; V is the volume of the test piece; k is a natural number to be determined in conjunction with dividing the range of a minimum measuring temperature $T_{min}$ to a maximum measuring temperature $T_{max}$, into n equal parts, in conformity to measurement conditions; and $M_k$ $(T_i)$ is a saturation magnetization at a measuring temperature $T_i$ in the chromium impoverished region having the chromium concentration $C_k$. The saturation magnetization is obtained in advance based on the following data (a), (b) and (c): (a) the relationship between saturation magnetization and chromium concentration at an absolute temperature of 0 (zero) K in the chromium impoverished region; (b) the relationship between Curie temperature and chromium concentration in the chromium impoverished region; and (c) the relationship between saturation magnetization and measuring temperature in the chromium impoverished region.

The present invention also provides an apparatus for detecting magnetic characteristics of the test piece for use in the above method. The apparatus comprises: a cooling-medium tank for containing a cooling medium; a test-piece housing disposed at the central region of the cooling-medium tank to receive the test piece therein; an exciting device mounted on the inner wall of the test-piece housing to excite the test piece; a support member for supporting the test piece in such a manner that it is located at the center position of the exciting device; a magnetic flux detector disposed around the test piece; a cooling device for supplying a cooling medium to the cooling-medium tank to circulatingly cool the test piece while allowing cooling gas generated from the cooling medium to flow into the test-piece housing; a heating device disposed below the test piece to heat the test piece; and means for controlling the measuring temperature of the test piece through the use of the cooling medium and the heating device.

While the present invention will be specifically described in connection with Inconel 600 alloy known as a typical one of chromium-containing nickel-based alloys, an object to be inspected by the method of the present invention is not limited to the Inconel 600 alloy. Inconel alloys include Inconel 600, Inconel 601, Inconel 625, Inconel 690 and Inconel 617. The Inconel 600 consists of about 74 wt% of nickel, about 16 wt% of chromium and about 10 wt% of iron. The term "chromium-containing nickel-based alloy" in the present invention means a nickel-based alloy containing chromium in an amount to the extent of causing the grain boundary precipitation of chromium carbide as in these Inconel alloys.

The Inconel 600 alloy has a Curie temperature (magnetic transition temperature) of 109 K. The mechanism of thermal sensitization and the temperature causing the thermal sensitization in the Inconel 600 alloy has been investigated through various researches. Heretofore, the verification of the thermal sensitization has been conducted based on etching using a chemical agent or electrochemical etching, and it has been reported that the thermal sensitization is accelerated at a temperature of 600 to 700°C.

In a chromium-containing nickel-based alloy, chromium carbide precipitates are formed in the vicinity of the crystal grain boundaries of the alloy, and the Curie temperature of a chromium impoverished region is dependent on the chromium concentration of the chromium impoverished region. FIG. 2 shows the relationship between Curie temperature and chromium concentration in a chromium impoverished region of the Inconel 600 alloy. While the gradient of the line illustrated in FIG. 2 is slightly changed at a chromium concentration of 14 wt%, the Curie temperature is increased approximately in proportion to decrease in the chromium concentration. The lowest chromium concentration in the previous researches is 6 wt%, which corresponds to a Curie temperature of 450 K.

In the inspection method of the present invention, a saturation magnetization at a measuring temperature $T_i$ in a chromium impoverished region having a chromium concentration $C_k$ is obtained in advance based on the above relationship between the Curie temperature and the chromium concentration, and a saturation magnetization of a test piece of chromium-containing nickel-based alloy is actually measured at each of a plurality of measuring temperatures defined by equally dividing a given measuring temperature range in the range of 109 K to 450 K or a minimum to a maximum of Curie temperatures corresponding to respective chromium concentrations in a chromium impoverished region of the alloy. Then, based on the pre-determined saturation magnetization and the actually measured saturation magnetization, an average spatial distribution of the chromium impoverished region of the test piece, or the chromium-concentration-specific volume of the chromium impoverished region adjacent to the grain boundaries of the test piece, is quantitatively determined according to a given calculation formula.

According to the method of the present invention, the volume of the chromium impoverished region of the chromium-containing nickel-based alloy adjacent to the crystal grain boundaries having chromium carbide precipitates formed during a welding process or high-temperature long-term use can be quantitatively determined in a divided manner on the basis of the chromium concentrations in the chromium impoverished region.

In addition, the variation in substantial chromium carbide precipitates of Inconel 600 structural member can be quantitatively determined in accordance with the respective calculated volumes $\nu_1$, $\nu_2$, - - - $\nu_n$ of the chromium impoverished region.

Furthermore, as to chemical heterogeneity which is one of factors causing stress corrosion cracking, for example, the volume of the chromium impoverished region having a chromium concentration of 10 wt% can be calculated to compute a probability of occurrence of stress corrosion cracking through simulation techniques or the like.

BRIEF DESCRIPTION OF DRAWINGS

FIG. 1 is a schematic diagram showing a chromium concentration distribution in chromium carbide precipitates and chromium-deficient portions adjacent to the crystal grain boundary of Inconel 600 alloy.

FIG. 2 is a graph showing the relationship between Curie temperature and chromium concentration in Inconel 600 alloy.

FIG. 3 is a graph showing one example of the distribution of the chromium-concentration-specific volume of a chromium impoverished region adjacent to the crystal grain boundaries of the Inconel 600 alloy.

FIG. 4 is a graph showing the relationship between saturation magnetization and chromium concentration at an absolute temperature of 0 (zero) K in the chromium impoverished region of the Inconel 600 alloy.

FIG. 5 is a graph showing the relationship between saturation magnetization and temperature (both the saturation magnetization and temperature are normalized) in the chromium impoverished region of the Inconel 600 alloy having a chromium concentration $C_{Cr}$ ranging from 14 wt% to 16 wt%.

FIG. 6 is a graph showing the relationship between saturation magnetization and temperature (both the saturation magnetization and temperature are normalized) in the chromium impoverished region of the Inconel 600 alloy having a chromium concentration $C_{Cr}$ ranging from 9 wt% to less than 14 wt%.

FIG. 7 is a graph showing the relationship between saturation magnetization and magnetic susceptibility in the Inconel 600 alloy.

FIG. 8 is a partially sectional conceptual diagram showing an apparatus for detecting magnetic characteristics of a test piece for use in implementing a method of the present invention.

FIG. 9 is a graph showing a magnetization curve of a test piece at each measuring temperature, wherein the test piece is prepared by aging Inconel 600 alloy at 700°C for 10 hours.

FIG. 10 is a graph showing one example of inspection results in which Inconel 600 alloy was subjected to aging at 700°C for each of 1 hour and 10 hours to produce a chromium impoverished region, and the distribution of the chromium-concentration-specific volume of the chromium impoverished region was actually determined through the method of the present invention.

FIG. 11 is a graph showing another example of inspection results in which Inconel 600 alloy was subjected to aging at 700°C for each of 10 hours and 100 hours to produce a chromium impoverished region, and the distribution of the chromium-concentration-specific volume of the chromium impoverished region was actually determined through the method of the present invention.

FIG. 12 is a graph showing still another example of inspection results in which the thickness d of the chromium impoverished region in FIG. 10 was determined with respect to each of the chromium concentrations.

FIG. 13 is a graph showing yet another example of inspection results in which the thickness d of the chromium impoverished region in FIG. 11 was determined with respect to each of the chromium concentrations.

BEST MODE FOR CARRYING OUT THE INVENTION

A process for determining the volume vk of a chromium impoverished region having a chromium concentration $C_k$ in a chromium-containing nickel-based alloy is programmed according to the following analysis procedure.

When Inconel 600 alloy as a typical one of chromium-containing nickel-based alloy has a chemically homogeneous composition free of the formation of carbide precipitates, the saturation magnetization of the alloy is zero at its Curie temperature (109 K) or more. In contrast, when a chromium impoverished region exists therein, the alloy has a certain saturation magnetization depending on a chromium concentration in the chromium impoverished region even at a temperature of 109 k or more. In cases where the saturation magnetization of a test piece is measured at a measuring temperature $T_i$ of 109 K or more, the saturation magnetization $M_s$ ($T_i$) can be determined according to the following calculation formula (1):

$$M_s(T_i) = \sum_{k=1}^{i} \frac{\nu_k M_k(T_i)}{V} \qquad (1)$$

In the above formula, νk is the volume of the chromium impoverished region having a chromium concentration $C_k$, and V is the volume of the test piece. k is a natural number to be to be determined in conjunction with dividing the range of a minimum measuring temperature $T_{min}$ to a maximum measuring temperature $T_{max}$, into n equal parts, in conformity to measurement conditions. A larger numerical value of n allows the distribution to be measured with a higher degree of accuracy. Conversely, an excessively small value of n permits only a rough analysis of the distribution. The minimum measuring temperature $T_{min}$ may be set at 105 K or 100 K, because the measurement may be fundamentally initiated at any temperature less than the Curie temperature of a chromium-containing nickel-based alloy. The maximum measuring temperature $T_{max}$ is preferably set at 450 K. If it is set at a room temperature of 300 K, a chromium concentration distribution ranging from 16 wt% to 9 wt% will be measured in the chromium impoverished region. The following description will be made in connection with the case where $T_{min}$ and $T_{max}$ are set at 100 K and a room temperature of 300 K, respectively.

$M_k$ ($T_i$) is a saturation magnetization at a measuring temperature $T_i$ in the chromium impoverished region having the chromium concentration $C_k$. The saturation magnetization is obtained in advance based on the following data (a), (b) and (c).

(a) The relationship between saturation magnetization and chromium concentration at an absolute temperature of 0 (zero) K in the chromium impoverished region
(b) The relationship between Curie temperature and chromium concentration in the chromium impoverished region
(c) The relationship between saturation magnetization and measuring temperature in the chromium impoverished region

More specifically, a saturation magnetization $M_k$ (0) of the chromium impoverished region with the chromium concentration $C_k$ at an absolute temperature of 0 (zero) K is first determined. Further, a Curie temperature $T_c$ K of the chromium impoverished region with the chromium concentration $C_k$ is determined. Then, according to the relationship (c) between a normalized saturation magnetization $M_k$ ($T_i$)/$M_k$ (0) and a normalized temperature $T_i$/$T_c$ K, a saturation magnetization $M_k$ ($T_i$)/$M_k$ (0) at the temperature $T_i$ is calculated. Based on this saturation magnetization $M_k$ ($T_i$)/$M_k$ (0) and the previously determined $M_k$ (0), a saturation magnetization $M_k$ ($T_i$) of the chromium impoverished region with the chromium concentration $C_k$ at the temperature $T_i$ can be obtained.

In this manner, $M_k$ ($T_i$) in the formula (1) is determined in advance. Then, a saturation magnetization $M_k$ ($T_i$) of a test piece at $T_i$ K is measured. Based on the pre-determined saturation magnetization and the measured saturation magnetization, the simultaneous equations of the formula (1) can be solved to obtain νk.

FIG. 3 shows the pre-determined values expressing the relationship between the volume νk and the chromium concentration $C_k$ in the chromium impoverished region of the Inconel 600 alloy. FIG. 4 shows the pre-determined values expressing the relationship between the saturation magnetization and the chromium concentration in the chromium impoverished region of the Inconel 600 alloy at an absolute temperature of 0 (zero) K. Further, FIG. 5 shows the relationship between a saturation magnetization (which is normalized by the saturation magnetization at an absolute temperature of 0 (zero) K) and each measuring temperature (which is normalized by the Curie temperature $T_c$) in one example where the chromium impoverished region of the Inconel 600 alloy has a chromium concentration of 14 wt% or more. In the same manner, FIG. 6 shows the relationship in another example where the chromium impoverished region has a chromium concentration of less than 14 wt%.

A saturation magnetization of the chromium impoverished region in the chromium concentration $C_k$ at an absolute temperature of 0 (zero) K can be obtained from FIG. 4. Further, a Curie temperature in the chromium concentration $C_k$ can be obtained from FIG. 2. Based on the ratio between this Curie temperature in the chromium concentration $C_k$ and the measuring temperature $T_i$, a saturation magnetization $M_k$ ($T_i$) at the measuring temperature $T_i$ in the chromium impoverished region with the chromium concentration $C_k$ is obtained using FIG. 5 or 6.

A measured value of the saturation magnetization $M_s$ ($T_i$) of the test piece at the measurement temperature $T_i$ is obtained by determining a magnetization curve. The magnetization curve at the measuring temperature $T_i$ has both a paramagnetic state and a ferromagnetic state. The ferromagnetic state arises from a region having a low chromium concentration and a Curie temperature of the measuring temperature $T_i$ or less.

In the formula (1), except for νk, all of the physical values are determined through the above process. The number of unknown values is n, and the number of formulas (1) is n correspondingly. Thus, the unknown values νk can be obtained by solving the simultaneous equations.

In order to obtain $\nu_1$, $\nu_2$, - - - $\nu_n$ through the above process, it is required to obtain an actually measured $M_s$ ($T_i$). In cases where $M_s$ ($T_i$) is obtained directly through the above process, a cooling medium and a heater are used to control the test piece to have each of the measuring temperatures, and a magnetic field is applied to the test piece from outside, for example, in the range of 0 to $2 \times 10^4$ Oe, to measure $M_s$ ($T_i$). The generation of a strong magnetic field involves a problem, such as increase in size of a measuring apparatus. In consideration of this point, a process for simply determining $M_s$ ($T_i$) under a weak magnetic field will be described below.

A magnetic susceptibility at the measuring temperature and a saturation magnetization have a simple relationship therebetween as expressed by the following formula (2):

$$\chi 0\ (T_i) = A\ M_s\ (T_i) \qquad (2),$$

wherein A is a proportionality multiplier.

For example, given that a magnetization by 50 Oe is defined as a magnetic susceptibility $\chi 0$, $\chi 0$ ($T_i$) and $M_s$ ($T_i$) are correlated with one another as shown in FIG. 7. As seen in FIG. 7, $\chi 0$ ($T_i$) and $M_s$ ($T_i$) have a strong correlation therebetween. The proportionality multiplier A is a constant almost independent of the measuring temperature and the chromium concentration. Thus, instead of directly measuring the saturation magnetization $M_s$ ($T_i$) at the measuring temperature Ti, the saturation magnetization $M_s$ ($T_i$) can be determined indirectly from the relationship of the formula (2) based on a magnetization measured under a weak magnetic field. In this case, the value of A is determined in advance.

The magnetization characteristic obtained by the above measurement at each of the measuring temperatures is based on magnetization under a weak magnetic field of 50 Oe, and thereby the saturation magnetization values of the test piece cannot be directly obtained. Thus, it is required to determine in advance a coefficient for obtaining an intended magnetization characteristic through a conventional magnetization measurement. However, this coefficient can be determined in advance by measuring the magnetization characteristic of a test piece identical to a known material having actually measured data.

The magnetic susceptibility value is determined from the pseudo-magnetization characteristic obtained in the above manner. Then, based on this value, the presence of substantial thermal sensitization in the test piece due to precipitation of chromium atoms can be verified, and then the quantity of the thermal sensitization can be determined.

In the above process, the constant A is dependent on the internal structure of a material. Thus, this constant A is obtained in advance using a preliminary-test piece having the same material as that of the actual test piece, and then the relationship between the magnetic susceptibility and volume fraction of the chromium impoverished region is determined according to the formula (2) using the obtained constant. This relationship allows the volume of the chromium impoverished region transformed by thermal sensitization to be readily determined correspondingly to the magnetic susceptibility obtained through the aforementioned measurement. Thus, any strong magnetic field is not required to obtain $\nu_1$, $\nu_2$, - - - $\nu_n$.

As above, according to the nondestructive inspection method of thermal sensitization in a structural member of Inconel 600 alloy, a magnetic susceptibility value is derived by the pseudo-magnetization curve obtained from an actual measurement to accurately determine the volume of the chromium impoverished region as shown in FIG. 10 without destruction of the structural member. In addition, through the comparison between the respective states of a specific material before and after thermally sensitized, an average spatial distribution of the chromium impoverished region due to thermal sensitization in a structural member of Inconel 600 alloy can be measured in a nondestructive manner. The above analysis can be readily performed using a pre-programmed computing unit.

FIG. 8 is a partially sectional conceptual diagram of a magnetic-characteristic detecting apparatus for use in implementing the method of the present invention. As shown in FIG. 8, a test-piece housing 2 is disposed at the central region of a cooling-medium tank 1, and an exciting device 3 composed of an electromagnet or a superconducting magnet is mounted on the inner wall of the housing 2. A cooling medium is supplied from a cooling device 4 to the cooling-medium tank 1 through a supply pipe 5. A cooling-gas supply pipe 6 is provided at the bottom wall of the test-piece housing 2 to allow gas generated from the cooling medium to flow into the test-piece housing 2. After cooling a test piece 8, the gas is circulated from the upper portion of the test-piece housing 2 to the cooling device 4 through a cooling-gas discharge pipe 7.

The test piece 8 is attached to a test-piece support member, and inserted into the housing 2 in such a manner that it is located at the central position of the exciting device 3. In FIG. 8, the test piece 8 is attached at the lower portion of a test-piece support rod 9 serving as the test-piece support member, and the test-piece support rod 9 is inserted from the center of the top wall of the test-piece housing 2 so as to allow the test piece 8 to be located at the central position of the exciting device 3. A magnetic flux detector 10 is attached to the test-piece support rod 9 in such a manner that it is located around the test piece 8. Measurement data from the magnetic flux detecting device 10 are transferred through a lead wire 11 to a computing unit 12 for analyzing a magnetization characteristic to calculate the volume of a chromium impoverished region.

A controller (not shown) is operable to supply an exciting current to the exciting device 3. A heating device 13 is disposed below the test piece 8 and close to the bottom wall of the test-piece housing 2 to control a measuring temperature of the test piece 8. A thermometer 14 is attached on the lower end of the support rod 9 to measure the temperature of the test piece 8. The cooling medium may be liquid nitrogen.

In a measurement using the above magnetic-characteristic detecting apparatus, the temperature of the test piece 8 is evenly controlled by a means (not shown) for controlling the measuring temperature of the test piece through the use of the cooling medium and the heating device 13. Then, the controller acts to supply an exciting current to the exciting device 3, and measurement data about voltage induced in the magnetic flux detector 10 in response to the exciting current are led to the computing unit 12. The computing unit amplifies and integrates the measurement data to obtain the magnetization characteristic of the test piece 8 at the measuring temperature, and the volume of the chromium impoverished region calculated according to an analysis program is displayed on a display unit 15. Subsequently, the measurement of magnetic characteristic will be repeatedly performed at each of the equally-divided measuring temperatures. Through the above operation, a saturation magnetization $M_k$ ($T_i$) of the test piece at each of the plurality of measuring temperatures defined by equally dividing a given measuring temperature range in the range of a minimum to a maximum of Curie temperatures corresponding to respective chromium concentrations in the chromium impoverished region can be measured.

[EXAMPLE]

Example 1

Inconel 600 alloy was subjected to aging at 700°C for 10 hours, and used as a test piece. The saturation magnetization of this test piece was measured using the inspection apparatus as shown in FIG. 9. Measuring temperatures were defined by equally dividing a temperature range of 100 to 300 K into 10 parts. A magnetization curve at the respective measuring temperatures was obtained as shown in FIG. 9. A magnetization curve of a saturation magnetization $M_s$ ($T_i$) at a measuring temperature Ti was extrapolated from the magnetization curve illustrated in FIG. 9, and the saturation magnetization $M_s$ ($T_i$) was obtained from the intersecting point between the extrapolated magnetization curve and the vertical axis (external magnetic field: zero). Then, the relationship between the measuring temperature Ti and the saturation magnetization $M_s$ ($T_i$) (unit: emu/g) was obtained as shown in Table 1.

Table 1

| Saturation magnetization at each temperature in Inconel 600 subjected to aging at 700°c for 10 hours | | | | | | | | | | | |
|---|---|---|---|---|---|---|---|---|---|---|---|
| Temperature | 100K | 120K | 140K | 160K | 180K | 200K | 220K | 240K | 260K | 280K | 300K |
| $M_s$ ($T_i$) | 11.8 | 8.42 | 5.23 | 2.75 | 1.27 | 0.58 | 0.271 | 0.13 | 0.067 | 0.037 | 0.015 |

$\nu k$ (k = 1, 2, 3, - - -, 10) was obtained by the following calculation.

As for $M_s$ (300), Ti =300 and k =1 can be assigned to the formula (1) to express $M_s$ (300) as the following formula (3):

$$M_s\ (300) = \nu_1\ M_1\ (300)/V \qquad (3)$$

A chromium impoverished region with a Curie temperature of 300 k or more has a chromium concentration of 10.2 wt% or less. Given that the chromium impoverished region with a Curie temperature of 300 k or more is represented by a chromium impoverished region with a Curie temperature of 350 k. The chromium impoverished region with a Curie temperature of 350 K has a chromium concentration of 8.82 wt% as can be read from FIG. 2, and then a saturation magnetization at an absolute temperature of 0 (zero) K is 38.3 emu/g as can be read from FIG. 4. A Curie temperature at a chromium concentration of 8.82 wt% is 350 K. As to a saturation magnetization at 300 K, a normalized saturation magnetization corresponding to 300/350 (= 0.86) is 0.52 as can be read from FIG. 6, and a saturation magnetization based on the pre-determined data is 38.3 × 0.52, or $M_1$ (300) = 19.9 emu/g. Then, $M_s$ (300) is 0.015 emu/g as can be read from Table 1. Thus, $\nu_1/V$ is determined as $7.5 \times 10^{-4}$.

In the same manner, the volume $\nu_2$ of the chromium impoverished region having a Curie temperature of 280 K to less than 300 K or a chromium concentration between 11.x wt% or less and 10 wt% or more can be determined according to the following formula (4):

$$M_s\ (280) = \{\nu_1\ M_1\ (280) + \nu_2\ M_2\ (280)\}\ /\ V \qquad (4)$$

$M_s$ (280) = 0.037 emu/g can be read from the actually measured value (Table 1). $\nu_1/V$ has been determined as $7.5 \times 10^{-4}$ in the previous operation. $M_1$ (280) is a saturation magnetization at a Curie temperature of 280 K in a chromium concentration of 8.82 wt%. Thus, a normalized saturation magnetization corresponding to 280/350 = 0.80 is 0.602 as can be read from FIG. 6, and a saturation magnetization based on the pre-determined data is $38.3 \times 0.602$ or 23.0 emu/g. Then, $M_2$ (280) is a saturation magnetization in a chromium concentration of 10.7 wt% (Curie temperature: 300 K). A saturation magnetization of $M_2$ (280) at an absolute temperature of 0 (zero) K is 33.8 emu/g as can be read from FIG. 4. Thus, a normalized corresponding to 280/300 (= 0.93) is 0.36 as can be read from FIG. 6, and a saturation magnetization based on the pre-determined data is $33.8 \times 0.36$. Based on this value, $M_2$ (280) at 280 K is determined as 12.2 emu/g. These values are assigned to the formula (4), and resulting $0.037 = 7.7 \times 10^{-4} \times 23.0 + 12.2\ \nu_2/V$ is transformed to $\nu_2/V = 1.58 \times 10^{-3}$ to obtain $\nu_2$.

The above operations is repeatedly performed to determine the values of other volume fractions $\nu_3/V$, $\nu_4/V$, - - -,$\nu_{10}/V$.

An actual Inconel 600 includes chromium precipitates even before aging. In consideration of this situation, the relationship between the chromium concentration and the volume of a chromium impoverished region adjacent to the crystal grain boundary of the alloy is determined as shown in FIGS. 10 and 11.

FIG. 10 shows an inspection result of a test piece having precipitates through aging at 700°C for each of 1 hour and 10 hours. FIG. 11 shows an inspection result of a test piece having precipitates through aging at 700°C for each of 10 hour and 100 hours. These results show the volume of the precipitates derived by subtracting a pre-aging volume from a post-aging volume. The result in FIG. 11 is the distribution of chromium precipitates in the vicinity of the grain boundaries calculated on the assumption that the chromium precipitates are formed in the crystal grain boundaries. The volume of a chromium impoverished region is increased dependent on the aging time, and the deficient region spreads over after 10 hours (FIG. 10). Then, chromium is supplied from around the deficient region to be back to normal (FIG. 11). This phenomenon corresponds to the result of optical microscopic observation.

Alternatively, the distribution of the chromium impoverished region adjacent to the crystal grain boundaries can be determined through a simple process based on the previously obtained $\nu_1$, $\nu_2$, $\nu_3$, - -, on the assumption that the crystal grains have a spherical shape. Specifically, given that each of the crystal grains has a spherical shape with a radius r and a volume V, and the thickness of the chromium impoverished region with a chromium concentration $C_k$ is d, the thickness d of the chromium impoverished region can be determined with respect to each of the chromium concentrations, because $\nu_k/V$ is obtained from the following formula: $\nu_k/V = (4\ \pi\ r^2\ d)\ /\ (4/3\ \pi\ r^3) = 3d/r$. The result is shown in FIGS. 12 and 13, wherein the horizontal axis represents the thickness (nm) of the chromium impoverished region on the assumption that a crystal grain boundary is 0 (zero) nm.

While the temperature range of 110 K to 300 K in the above example has been divided into 10 parts, the temperature range may be appropriately set up depending on requited information. Further, the temperature range may be equally subdivided to obtain information about the concentration distribution in the chromium impoverished region more sensitively.

INDUSTRIAL APPLICABILITY

According to the present invention, a saturation magnetization is directly measured using a magnetization measuring apparatus or indirectly obtained using a measured magnetic susceptibility, so as to determine the volume of a chromium impoverished region in a chromium-containing nickel-based alloy in a divided manner on the basis of chromium concentrations of the alloy, in accordance with the above actually measured saturation magnetization and a pre-determined saturation magnetization based on preliminary obtained data about the chromium impoverished region.

Thus, the present invention allows the level of thermal sensitization in a structure made of a chromium-containing nickel-based alloy typified by Inconel 600, such as nuclear reactors, or power generators for thermal power plants, to be reliably inspected in a nondestructive manner before occurrence of cracks due to grain-boundary attack cracking. In addition, the inspection can be performed using a simple magnetic-characteristic detecting apparatus equipped with a small-size cooling device.

A nondestructive inspection cannot be achieved by any etching-based inspection method irrespective of type, such as chemical or electrochemical. Moreover, the conventional method based on electrochemical etching can obtain poor information about the chromium impoverished region only in the surface of a test piece. In contrast, a magnetic method can obtain average information about the entire test piece including the surface and inside thereof. The method of the present invention is superior to the conventional electrochemical-etching-based method in terms of measurement accuracy.

## Claims

**1.** A method for nondestructive inspection of grain-boundary attack due to thermal sensitization in a chromium-containing nickel-based alloy, comprising:

measuring a saturation magnetization $M_s(T_i)$ of a test piece at each of a plurality of measuring temperatures defined by equally dividing a given measuring temperature range in the range of a minimum to a maximum of Curie temperatures corresponding to respective chromium concentrations in a chromium impoverished region of said alloy; and
calculating νk according to the following formula (1) to quantitatively determine the volume of the chromium impoverished region in a divided manner on the basis of the chromium concentrations:

$$M_s(T_i) = \sum_{k=1}^{i} \frac{\nu_k M_k(T_i)}{V} \qquad (1),$$

wherein: νk is the volume of the chromium impoverished region having a chromium concentration $C_k$;
V is the volume of said test piece;
k is a natural number to be determined in conjunction with dividing the range of a minimum measuring temperature $T_{min}$ to a maximum measuring temperature $T_{max}$, into n equal parts, in conformity to measurement conditions; and
$M_k$ ($T_i$) is a saturation magnetization at a measuring temperature $T_i$ in the chromium impoverished region having the chromium concentration $C_k$, said saturation magnetization being obtained in advance based on the following data (a), (b) and (c):

(a) the relationship between saturation magnetization and chromium concentration at an absolute temperature of 0 (zero) K in the chromium impoverished region;
(b) the relationship between Curie temperature and chromium concentration in the chromium impoverished region; and
(c) the relationship between saturation magnetization and measuring temperature in the chromium impoverished region.

**2.** An apparatus for detecting magnetic characteristics of the test piece for use in the method as defined in claim 1, comprising:

a cooling-medium tank for containing a cooling medium;
a test-piece housing disposed at the central region of said cooling-medium tank to receive said test piece therein;
an exciting device mounted on the inner wall of said test-piece housing to excite said test piece;
a support member for supporting said test piece in such a manner that it is located at the center position of said exciting device;
a magnetic flux detector disposed around said test piece;
a cooling device for supplying a cooling medium to said cooling-medium tank to circulatingly cool said test piece while allowing cooling gas generated from said cooling medium to flow into said test-piece housing;
a heating device disposed below said test piece to heat said test piece; and
means for controlling the measuring temperature of said test piece through the use of said cooling medium and said heating device.

FIG.1

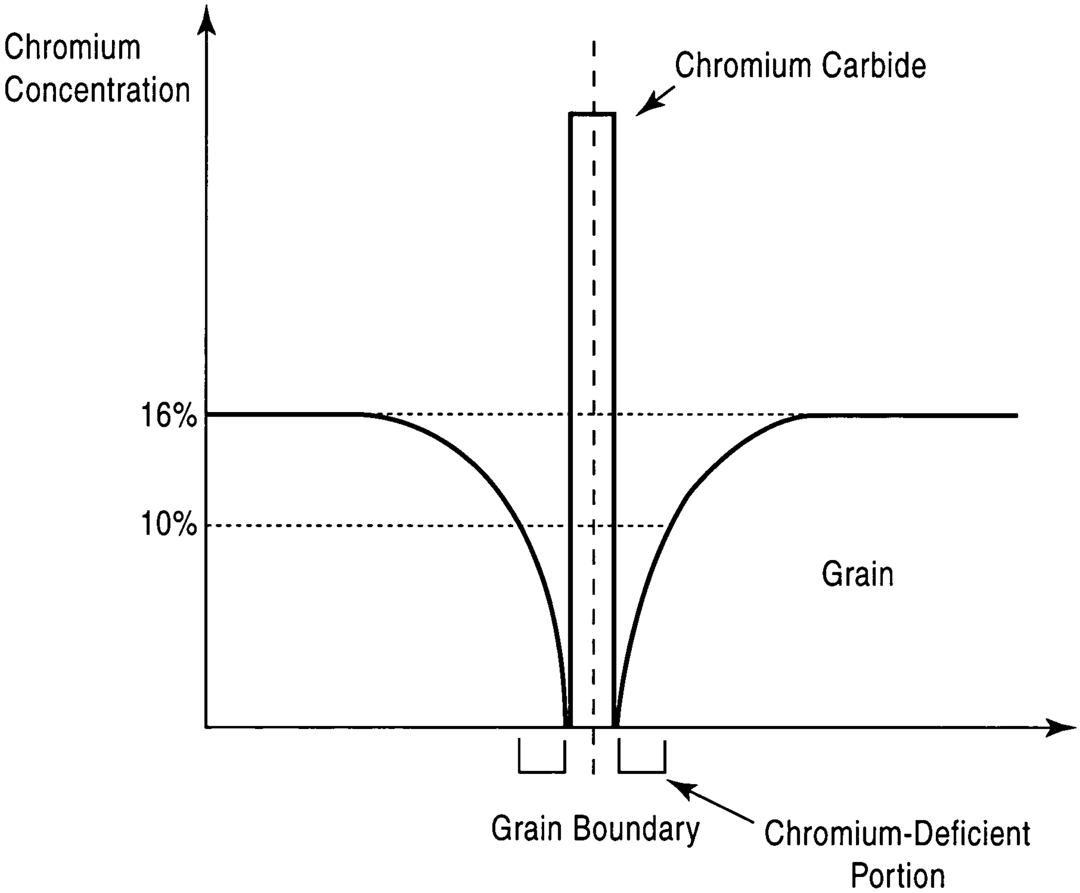
Chromium
Concentration
Chromium Carbide
16%
10%
Grain
Grain Boundary
Chromium-Deficient
Portion

FIG.2

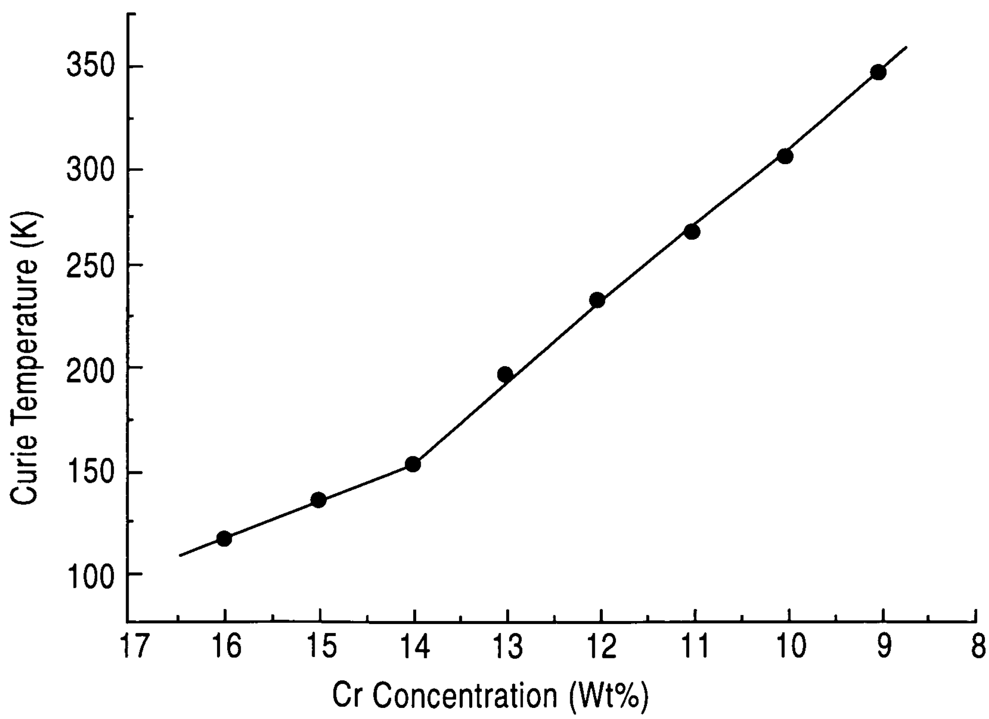
350
300
250
200
150
100
Curie Temperature (K)
17
16
15
14
13
12
11
10
9
8
Cr Concentration (Wt%)

FIG.3

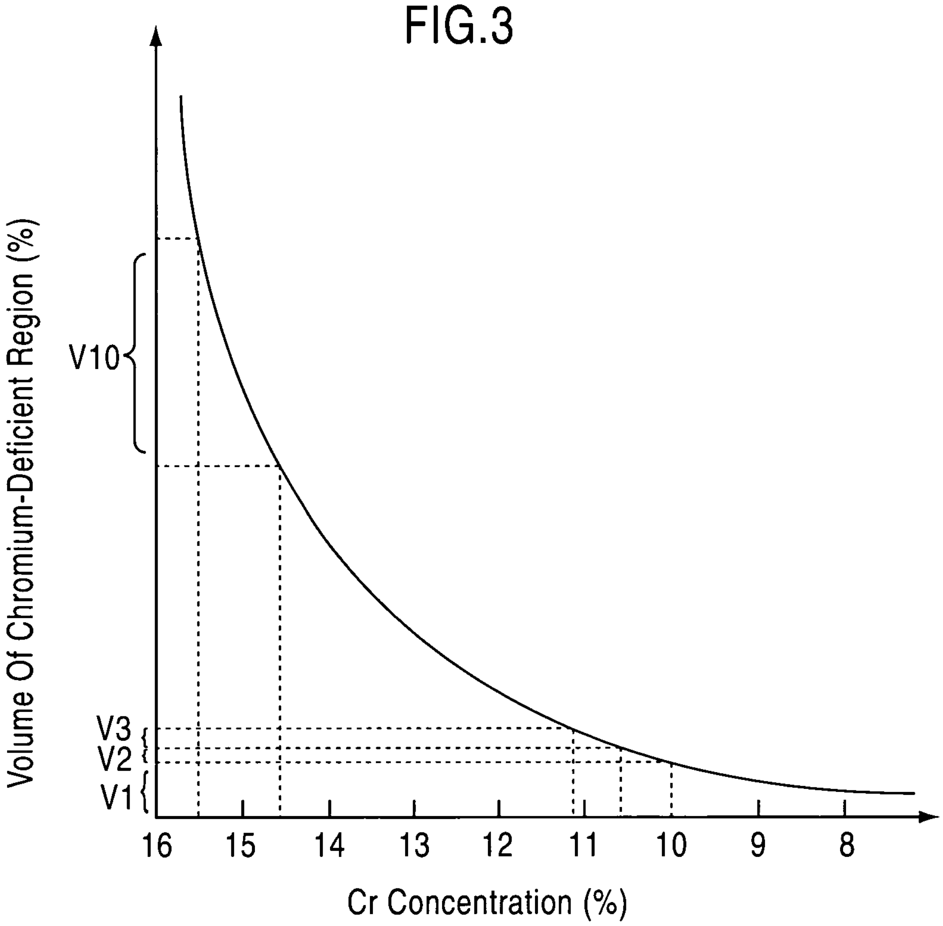
Volume Of Chromium-Deficient Region (%)
V10
V3
V2
V1
16
15
14
13
12
11
10
9
8
Cr Concentration (%)

FIG.4

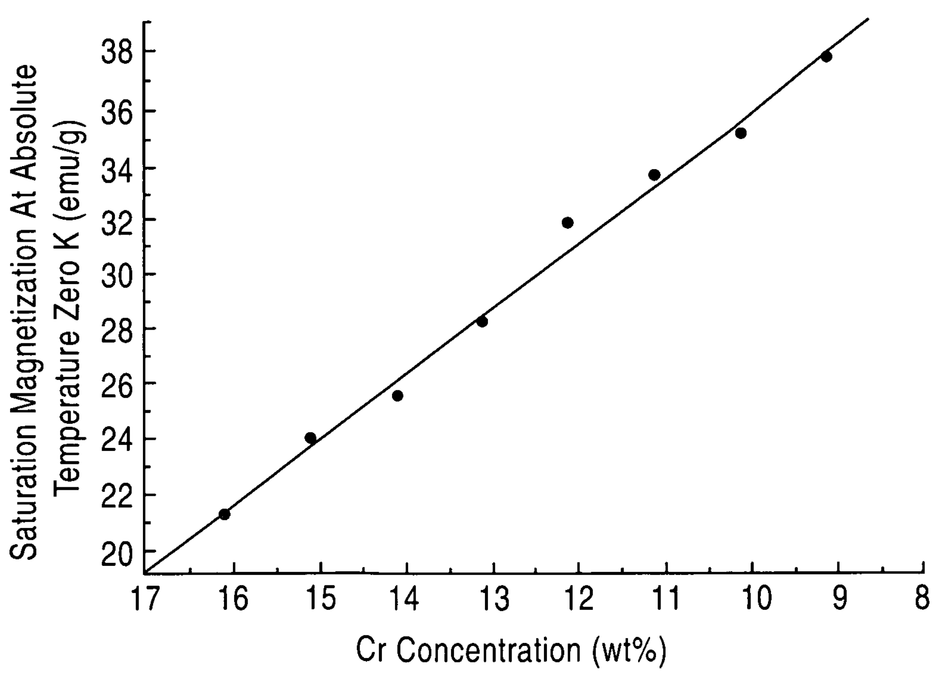
Saturation Magnetization At Absolute Temperature Zero K (emu/g)
38
36
34
32
30
28
26
24
22
20
17
16
15
14
13
12
11
10
9
8
Cr Concentration (wt%)

FIG.5

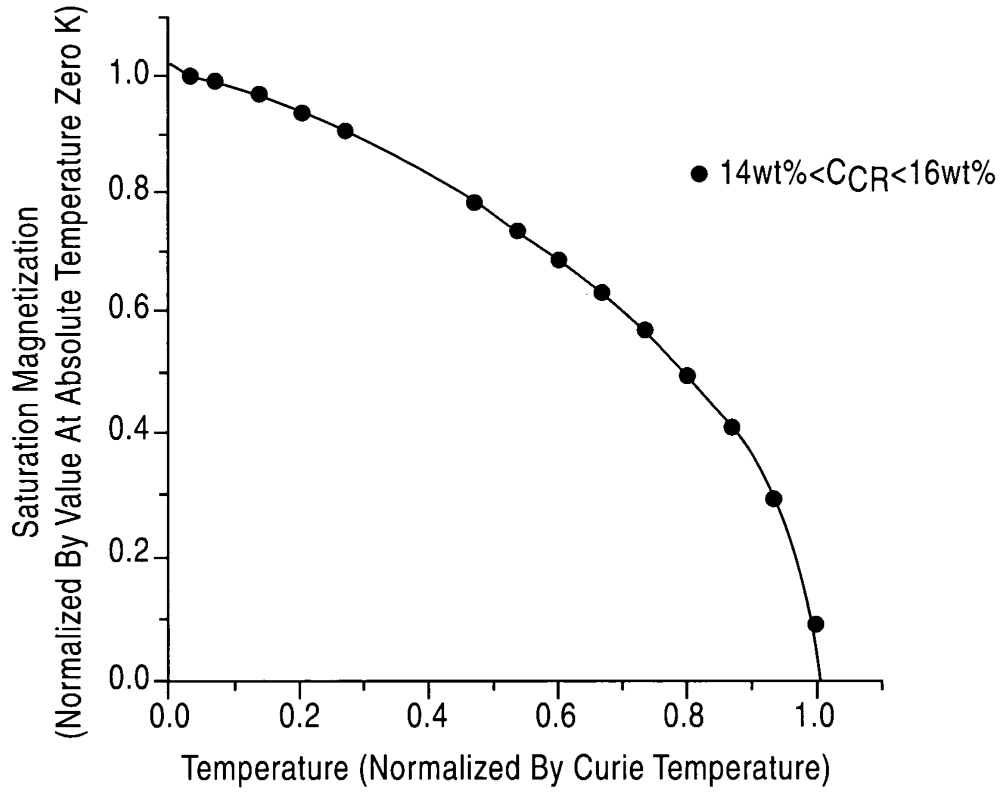
Saturation Magnetization
(Normalized By Value At Absolute Temperature Zero K)
1.0
0.8
0.6
0.4
0.2
0.0
0.0
0.2
0.4
0.6
0.8
1.0
Temperature (Normalized By Curie Temperature)
14wt%<CCR<16wt%

FIG.6

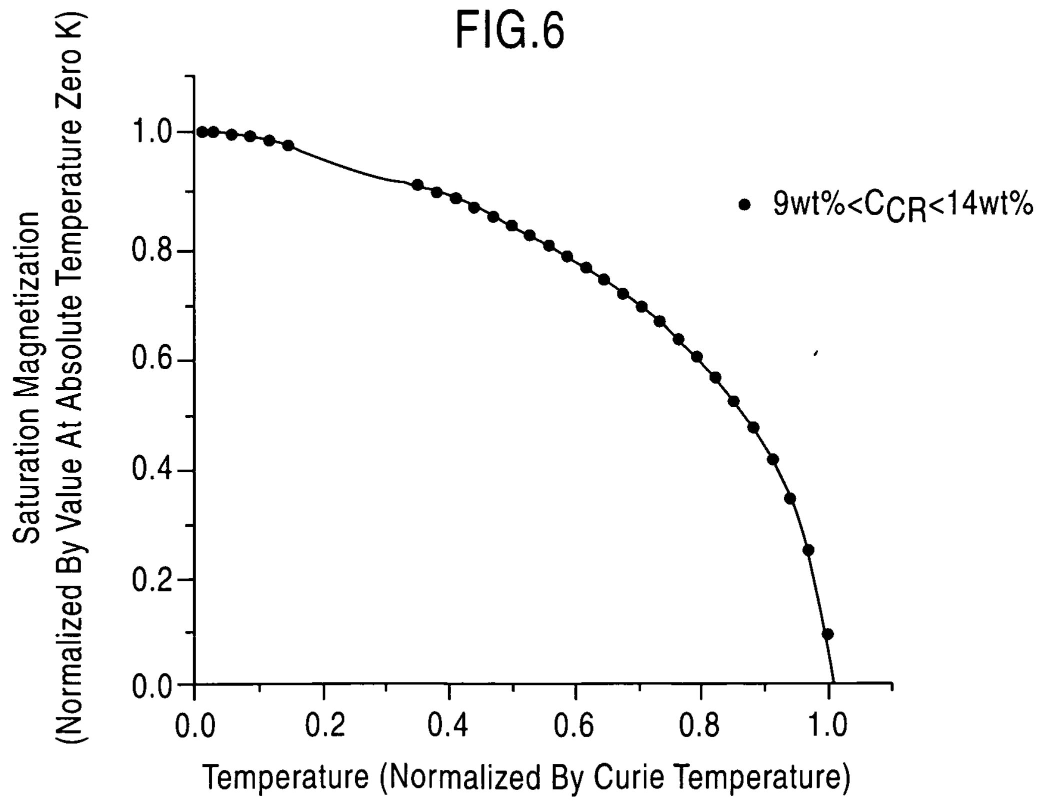
Saturation Magnetization
(Normalized By Value At Absolute Temprature Zero K)
1.0
0.8
0.6
0.4
0.2
0.0
0.0
0.2
0.4
0.6
0.8
1.0
Temperature (Normalized By Curie Temperature)
9wt%<CCR<14wt%

FIG.7

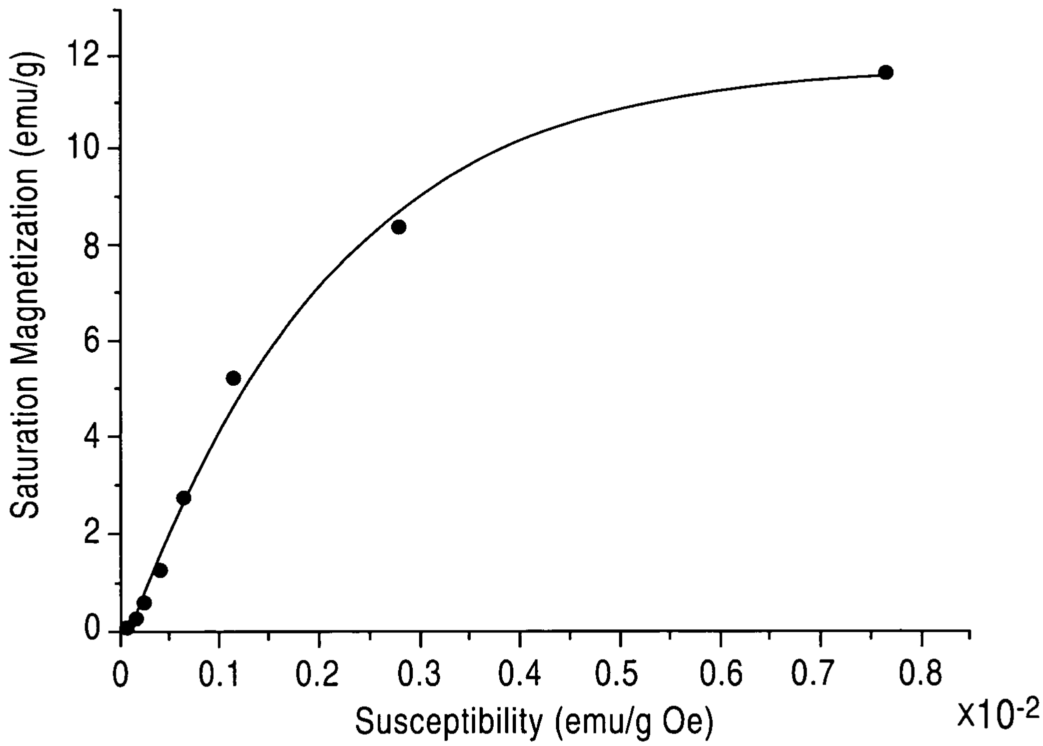

Saturation Magnetization (emu/g)
0
2
4
6
8
10
12
Susceptibility (emu/g Oe)
0
0.1
0.2
0.3
0.4
0.5
0.6
0.7
0.8
x10-2

FIG.8

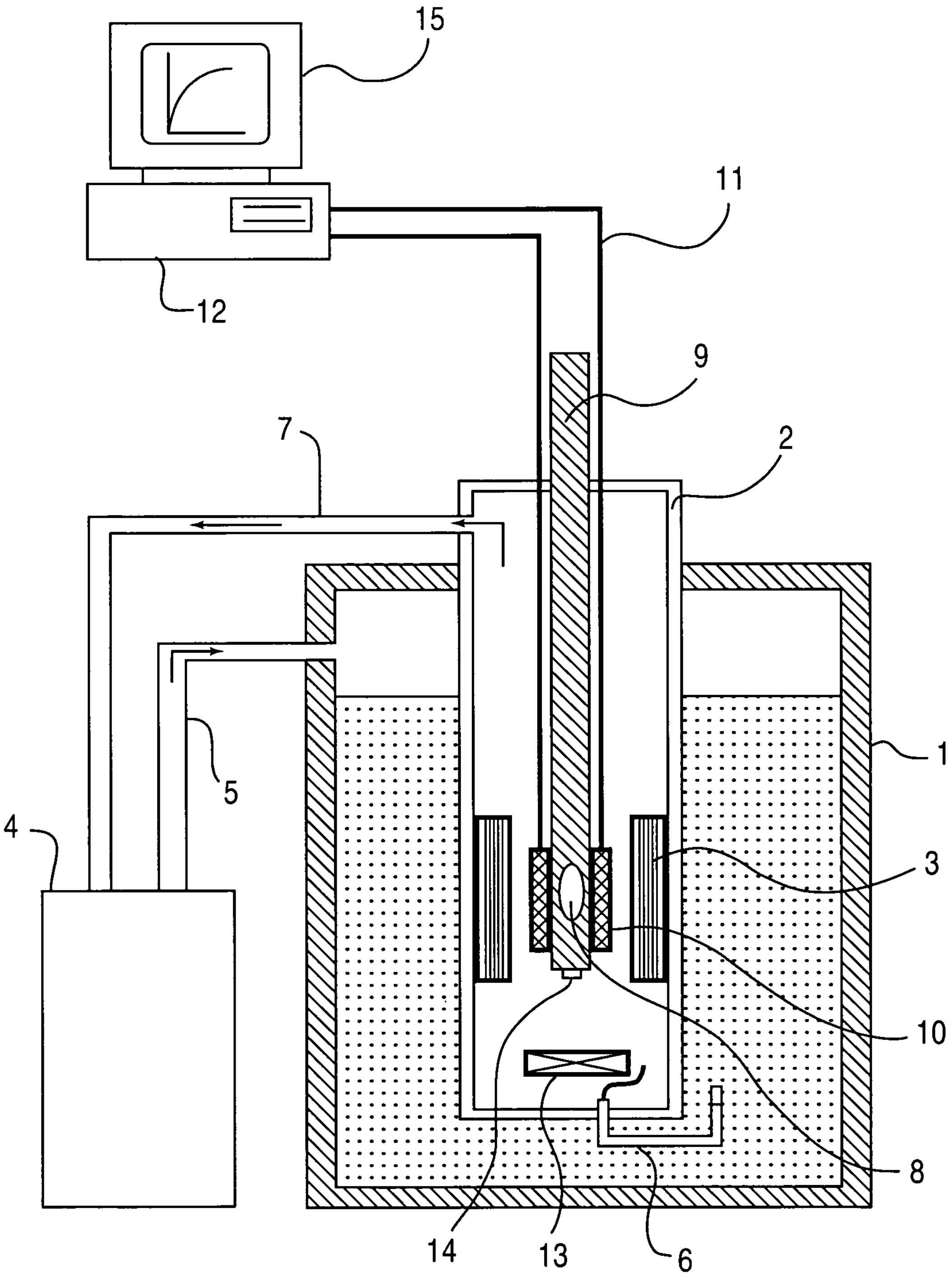

FIG.9

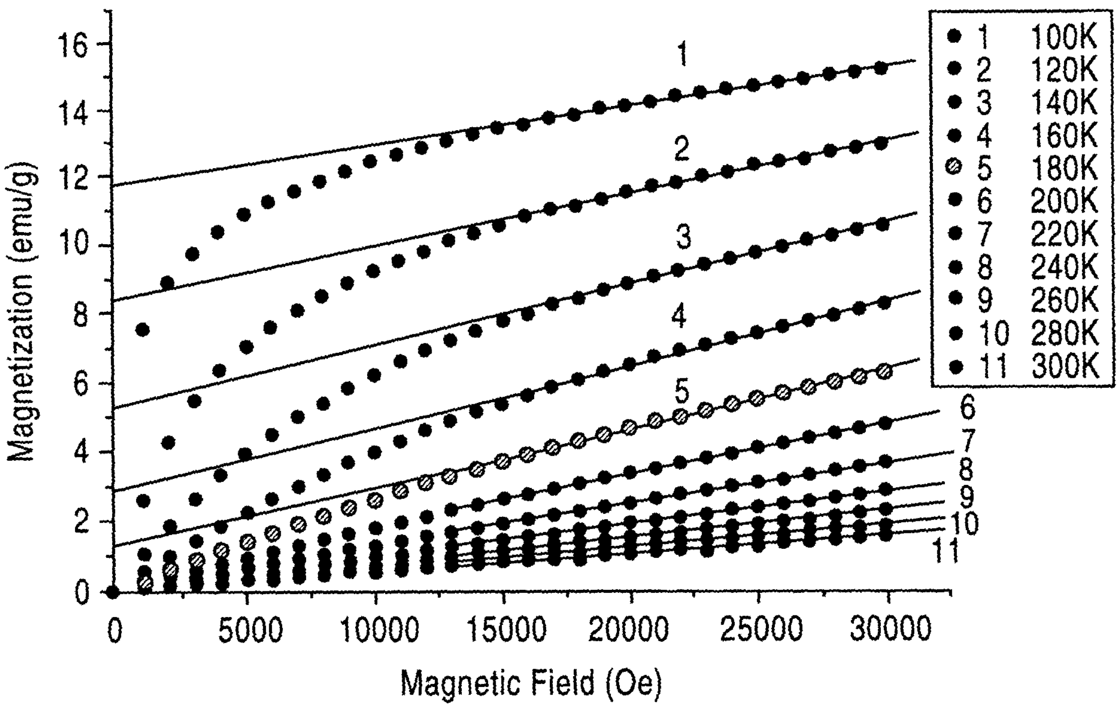
Magnetization (emu/g)
Magnetic Field (Oe)
1 100K
2 120K
3 140K
4 160K
5 180K
6 200K
7 220K
8 240K
9 260K
10 280K
11 300K

FIG.10

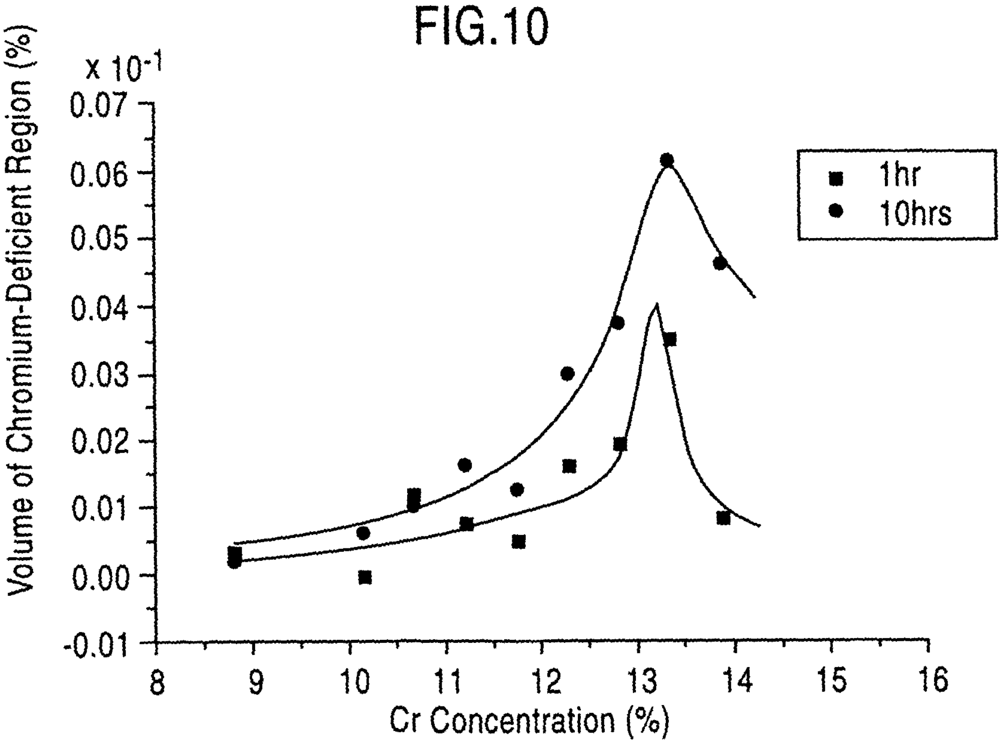
Volume of Chromium-Deficient Region (%)
x 10-1
Cr Concentration (%)
1hr
10hrs

FIG.11

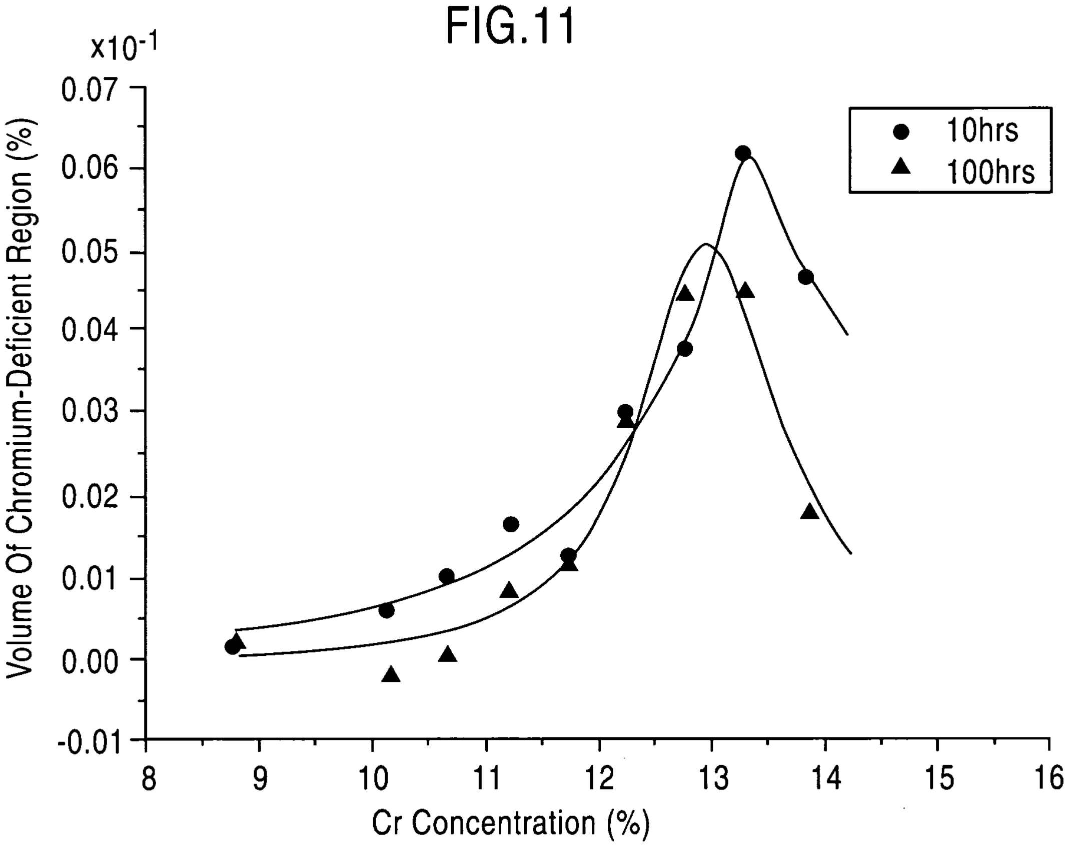
x10^-1
0.07
0.06
0.05
0.04
0.03
0.02
0.01
0.00
-0.01
Volume Of Chromium-Deficient Region (%)
8
9
10
11
12
13
14
15
16
Cr Concentration (%)
10hrs
100hrs

FIG.12

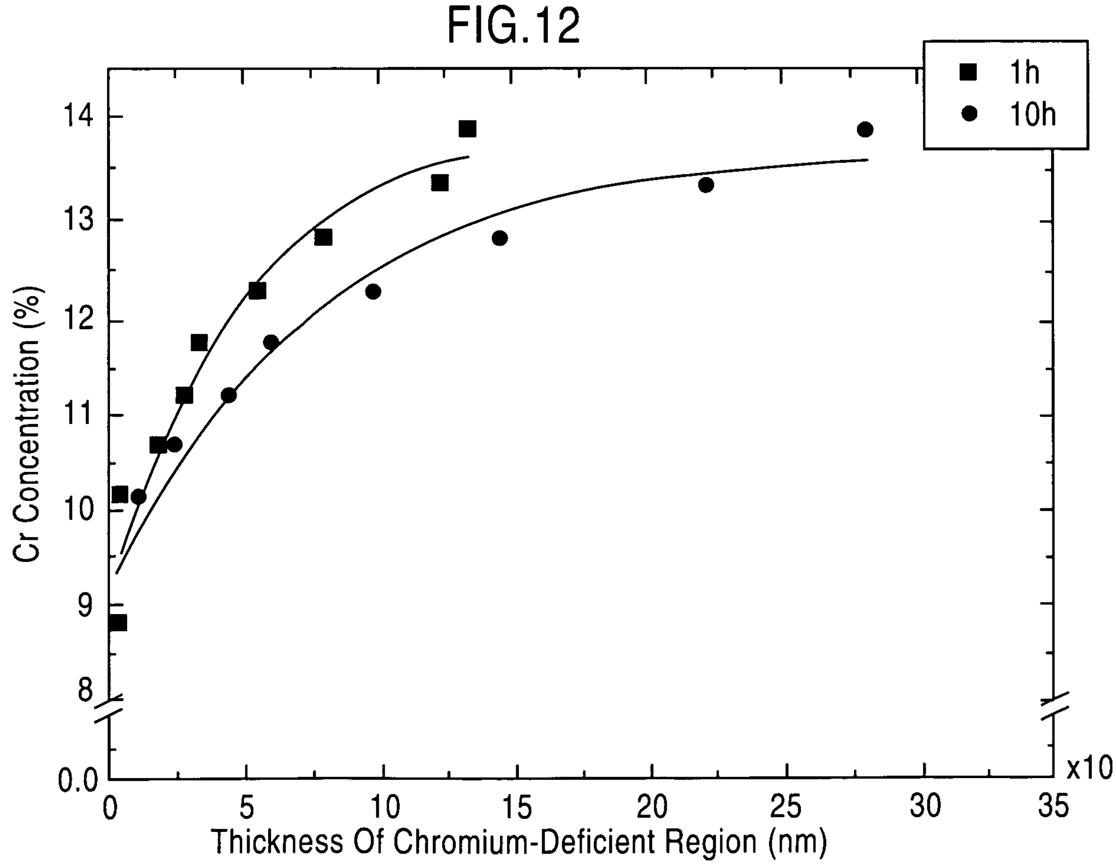
14
13
12
11
10
9
8
0.0
Cr Concentration (%)
0
5
10
15
20
25
30
35
x10
Thickness Of Chromium-Deficient Region (nm)
1h
10h

FIG.13

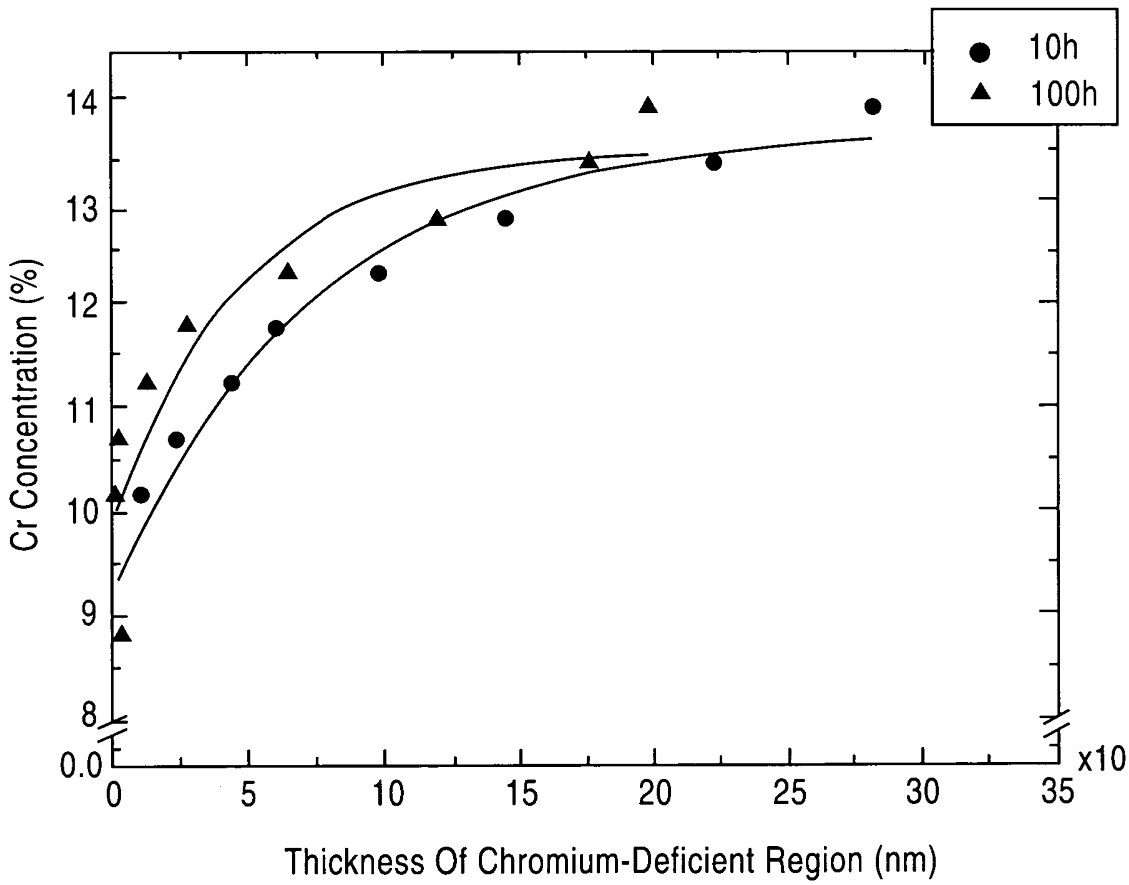
10h
100h
14
13
12
11
10
9
8
0.0
Cr Concentration (%)
0
5
10
15
20
25
30
35
x10
Thickness Of Chromium-Deficient Region (nm)

## INTERNATIONAL SEARCH REPORT

International application No.
PCT/JP03/04581

A. CLASSIFICATION OF SUBJECT MATTER
Int.Cl[7] G01N27/72

According to International Patent Classification (IPC) or to both national classification and IPC

B. FIELDS SEARCHED

Minimum documentation searched (classification system followed by classification symbols)
Int.Cl[7] G01N27/72-27/90

Documentation searched other than minimum documentation to the extent that such documents are included in the fields searched

| | | | |
|---|---|---|---|
| Jitsuyo Shinan Koho | 1922-1996 | Toroku Jitsuyo Shinan Koho | 1994-2003 |
| Kokai Jitsuyo Shinan Koho | 1971-2003 | Jitsuyo Shinan Toroku Koho | 1996-2003 |

Electronic data base consulted during the international search (name of data base and, where practicable, search terms used)
JOIS[RYUKAIFUSHOKU, NETSUEIBINKA, KUROMU, HOWAJIKA](in Japanese)
WPI[intergranular corrosion, thermally sensitize, chrome, saturation magnetization]

C. DOCUMENTS CONSIDERED TO BE RELEVANT

| Category* | Citation of document, with indication, where appropriate, of the relevant passages | Relevant to claim No. |
|---|---|---|
| A | JP 55-42060 A (Sumitomo Metal Industries, Ltd.), 25 March, 1980 (25.03.80), Full text; Figs. 1 to 4 (Family: none) | 1,2 |
| A | JP 54-156697 A (Mitsubishi Heavy Industries, Ltd.), 10 December, 1979 (10.12.79), Full text; Figs. 1 to 4 (Family: none) | 1,2 |
| A | JP 2001-141700 A (Osaka Gas Co., Ltd.), 25 May, 2001 (25.05.01), Full text; Figs. 1 to 16 (Family: none) | 2 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 11 June, 2003 (11.06.03) | 24 June, 2003 (24.06.03) |
| Name and mailing address of the ISA/ Japanese Patent Office | Authorized officer |
| Facsimile No. | Telephone No. |

Form PCT/ISA/210 (second sheet) (July 1998)

**INTERNATIONAL SEARCH REPORT**

| International application No. |
|---|
| PCT/JP03/04581 |

C (Continuation). DOCUMENTS CONSIDERED TO BE RELEVANT

| Category* | Citation of document, with indication, where appropriate, of the relevant passages | Relevant to claim No. |
|---|---|---|
| A | Microfilm of the specification and drawings annexed to the request of Japanese Utility Model Application No. 170482/1988(Laid-open No. 91980/1990) (Sumitomo Heavy Industries, Ltd.), 20 July, 1990 (20.07.90), Full text; Figs. 1 to 2 (Family: none) | 2 |

Form PCT/ISA/210 (continuation of second sheet) (July 1998)